# EUROPÄISCHE PATENTSCHRIFT

(11) **EP 1 738 768 B1**
(45) Veröffentlichungstag und Bekanntmachung des Hinweises auf die Patenterteilung: **20.05.2009**
(21) Anmeldenummer: 06012204.1
(22) Anmeldetag: 14.06.2006
(51) Int. Cl.: A61K 47/18, A61K 47/12, A61K 9/08, A61K 31/58, A61P 21/02

(54) **Wässrige Formulierung für Vecuroniumbromid**
Aqueous formulation for vecuronium bromide
Préparation aqueuse pour bromure de vecuronium

(30) Priorität: 30.06.2005 DE 102005030423
(43) Veröffentlichungstag der Anmeldung: 03.01.2007
(73) Patentinhaber: Inresa Arzneimittel GmbH, 79114 Freiburg i. Br. (DE)
(72) Erfinder: Harrant, Gernot, 67000 Strasbourg (FR)
(74) Vertreter: Stoppkotte, Cornelia

(56) Entgegenhaltungen:
- EP-A- 0 707 853
- WO-A-01/45741
- US-A- 5 681 573
- TRISSEL: "Handbook on Injectable Drugs" AMERICAN SOCIETY OF HEALTH-SYSTEM PHARMACISTS (ASHP) * Seite 1286 - Seite 1287 *

## Beschreibung

Die vorliegende Erfindung betrifft eine neue und verbesserte pharmazeutische Formulierung für Vecuroniumbromid in Form einer wässrigen Lösung gemäß Patentanspruch 1 sowie ein Verfahren zur Herstellung einer solchen Lösung gemäß Patentanspruch 9.

Vecuroniumbromid (INN) (1-(3α,17β-Diacetoxy-2β-piperidino-5α-androstan-16β-yl)-1-methyl-piperidiniumbromid bzw. 1-[(2β,3α,5α,16β,17β)-3,17-Bis(acetyloxy)-2-(1-piperidinyl)androstan-16-yl]-1-methylpiperidiniumbromid) ist ein bekanntes nicht depolarisierendes, peripheres Muskelrelaxans, das vor allem im Bereich der Klinik eingesetzt wird, um vor Operationen eine Relaxation der Skelettmuskeln zu erreichen. Es wird aber auch in der Notfallmedizin, beispielsweise in Rettungswagen eingesetzt. Vecuroniumbromid wird dabei üblicherweise intravenös verabreicht.

Vecuroniumbromid weist die folgende chemische Strukturformel auf:

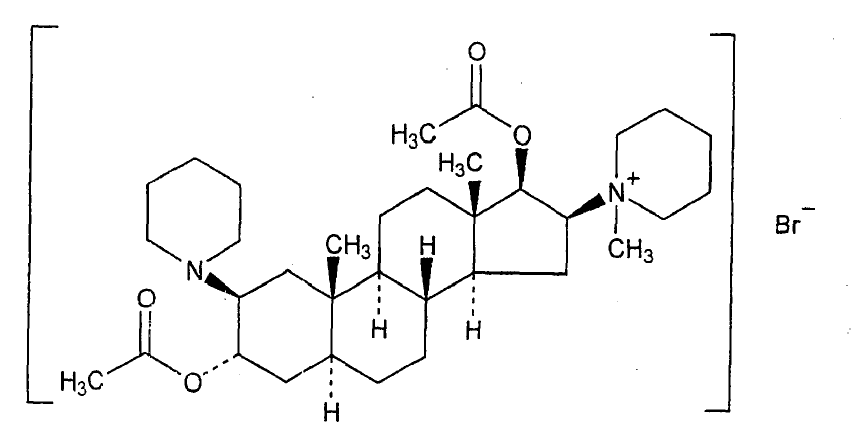

Vecuroniumbromid wird beispielsweise in dem europäischen Patent Nr. 0 008 824 B1 (Akzo N.V.) beschrieben. In dieser Patentschrift wird angegeben, dass sich Vecuroniumbromid in Wasser sehr schnell zersetzt, so dass wässrige Lösungen für Injektionszwecke nicht geeignet sind. Dagegen wurde gefunden, dass Säureadditionssalze von Vecuroniumbromid in wässriger Lösung deutlich stabiler sind. Als bevorzugte Säuren zur Stabilisierung durch die Bildung von Säureadditionssalzen werden in diesem Dokument z.B. Salzsäure, Bromwasserstoffsäure, Maleinsäure, Citronensäure, Phosphorsäure und Weinsäure genannt. Weiterhin wird beschrieben, dass die Säureadditionssalze lyophilisiert werden können.

Bei dem verbreitet verwendeten Vecuroniumbromidpräparat Norcuron^{®}, das unter das obige Patent fällt, handelt es sich um ein solches lyophilisiertes Präparat, das auf einem Säureadditionssalz von Vecuroniumbromid basiert.

In dem späteren Europäischen Patent Nr. 0 682 522 B1 (N.V. Inpharm et al.) wird ein Verfahren zur Herstellung eines Lyophilisats von Vecuroniumbromid beschrieben, das die Herstellung eines Säureadditionssalzes überflüssig macht. Dieses Lyophilisat wird dann zur Herstellung einer Injektionslösung in einer Aminosäurelösung mit physiologischem pH oder in einem pharmazeutisch verträglichen Puffersystem bei pH 7 bis 7,4 gelöst. Allerdings sind die so hergestellten Lösungen nicht lange haltbar. So beträgt der Wirkstoffgehalt der beschriebenen Lösungen nach 8 Stunden nur noch 95 bis 97%.

Darüber hinaus beschreibt das Europäische Patent Nr. 0 707 853 B1 (Poli Industria Chimica S.p.A.) ein pharmazeutisches Präparat zur parenteralen Verabreichung, das eine stabilisierte Lösung eines steroiden, neuromuskulären Blockierungsmittels zusammen mit einer zwitterionischen Substanz umfasst, welche einen isoelektrischen Punkt von nicht höher als 7 hat, was ebenfalls die Herstellung von Säureadditionssalzen überflüssig macht. Das steroide, neuromuskuläre Blockierungsmittel kann unter anderem Vecuroniumbromid sein. Auch in diesem Dokument werden die beschriebenen Lösungen überwiegend lyophilisiert, können aber auch in Form von gebrauchsfertigen Lösungen vorliegen. Diese Lösungen sind aber ebenfalls nur sehr begrenzt haltbar.

Wie sich aus dem oben beschriebenen Stand der Technik ergibt, sind gut haltbare wässrige Lösungen von Vecuroniumbromid bisher nicht verfügbar, so dass Vecuroniumbromid derzeit praktisch nur als Lyophilisat eingesetzt wird, das unmittelbar vor der Anwendung in Lösung gebracht werden muss. Die Herstellung von Lyophilisaten ist allerdings aufwendig und teuer und hat insbesondere den Nachteil, dass die zu injizierende Lösung jedes Mal vor der Verabreichung frisch hergestellt werden muss. Dieses ist insbesondere beim Einsatz in Rettungswagen nachteilig.

Ausgehend von diesem Stand der Technik ist es Aufgabe der vorliegenden Erfindung, eine stabile und besser haltbare wässrige Lösung von Vecuroniumbromid bereitzustellen, welche die Herstellung von Lyophilisaten überflüssig macht.

Diese Aufgabe wird gelöst durch ein pharmazeutisches Präparat nach Patentanspruch 1 sowie ein Verfahren zur Herstellung eines solchen Präparats nach Patentanspruch 9.

Der gegenwärtige Erfinder hat überraschend gefunden, dass eine stabilere wässrige Lösung von Vecuroniumbromid erhalten werden kann, wenn eine Aminosäure mit einem isoelektrischen Punkt größer 7 und mindestens eine Säure zur Einstellung des pH-Wertes auf einen Wert in dem Bereich von ca. 3 bis 5 in der wässrigen Lösung vorliegen.

Als Aminosäure mit einem isoelektrischen Punkt größer 7 wird Arginin (pI = 11,15), Lysin (pI = 9,59) oder Histidin (pI = 7,47) oder eine beliebige Kombination von diesen verwendet, wobei Arginin besonders bevorzugt ist. Diese Aminosäuren liegen vorzugsweise in einer Konzentration von ca. 0,05 bis 0,2 Mol/Liter in dem wässrigen Präparat vor.

Als Säure zur Einstellung des pH-Wertes auf einen Wert in dem Bereich von 3 bis 5 werden eine Aminosäure mit einem isoelektrischen Punkt kleiner 3,5 und eine organische Säure eingesetzt. Diese liegt besonders bevorzugt in einer Konzentration von 0,01 bis 0,5 Mol/Liter, noch bevorzugter von 0,05 bis 0,2 Mol/Liter in dem wässrigen Präparat vor. Werden mehrere Säuren eingesetzt, so liegt die Gesamtkonzentration der Summe dieser Säuren vorzugsweise ebenfalls in dem oben genannten Bereich.

Als Aminosäuren mit einem isoelektrischen Punkt kleiner 3,5 sind insbesondere Asparaginsäure (auch Aspartinsäure genannt) (pI = 2,77) oder Glutaminsäure (pI = 3,22) geeignet. Dabei ist die Konzentration der Aminosäure mit einem isoelektrischen Punkt kleiner 3,5 besonders bevorzugt ungefähr äquimolar zu der Konzentration der Aminosäure mit einem isoelektrischen Punkt größer 7. In diesem Fall kann der pH-Wert, falls erforderlich, mit einer weiteren organischen Säure eingestellt werden.

Beispiele für organische Säuren, die zur Einstellung des pH-Wertes eingesetzt werden können, sind Citronensäure, Essigsäure, Propionsäure, Maleinsäure, Fumarsäure, Bernsteinsäure, Glutarsäure, Weinsäure oder Milchsäure bzw. eine beliebige Kombination von diesen. Citronensäure ist hierbei als vielfach im pharmazeutischen Bereich eingesetzte physiologische Säure besonders geeignet.

Der gegenwärtige Erfinder hat festgestellt, dass die erhaltenen pharmazeutischen Präparate besonders stabil und haltbar sind, wenn diese einen pH-Wert von 3 bis 5, insbesondere 3,5 bis 4,5, vorzugsweise 3,9 bis 4,1 und besonders bevorzugt ca. 4,0 aufweisen.

Vecuroniumbromid liegt in den erfindungsgemäßen Präparaten insbesondere in einer Konzentration von 1 - 5 mg/ml, noch bevorzugter 2 - 4 mg/ml vor.

Ein besonders bevorzugtes pharmazeutisches Präparat wird dann erhalten, wenn als Aminosäure mit einem isoelektrischen Punkt größer 7 Arginin und als Säuren zur Einstellung des pH-Wertes Asparaginsäure und Citronensäure verwendet werden. Noch bevorzugter liegt die Asparaginsäure dabei ungefähr äquimolar zu dem Arginin vor. Dieses Präparat ist besonders stabil, wenn der pH-Wert des Präparats 3,9 bis 4,1, insbesondere ca. 4,0 beträgt.

Die erfindungsgemäßen Präparate können unmittelbar eingesetzt werden, d. h. sie liegen in Form von gebrauchsfertigen Lösungen vor.

Nach Wunsch können die erfindungsgemäßen Präparate zusätzlich weitere pharmazeutisch verträgliche Zusatzstoffe wie z.B. Konservierungsmittel enthalten.

Das pharmazeutische Präparat gemäß der Erfindung wird durch das im Folgenden beschriebene Verfahren hergestellt, welches die folgenden Schritte umfasst:
(a) Zugeben einer Aminosäure mit einem isoelektrischen Punkt größer 7 zu Wasser und Auflösen derselben unter Rühren,
(b) Zugeben mindestens einer Säure zur Einstellung des pH-Wertes auf einen Wert in dem Bereich von ca. 3 bis 5 zu der Lösung aus Schritt (a) und Weiterrühren bis zu deren Auflösung,
(c) Zugeben von Vecuroniumbromid und Weiterrühren bis zu dessen Auflösung und
(d) falls erforderlich erneutes Zugeben einer Säure zur Einstellung des pH-Wertes in dem Bereich von 3 bis 5.

Das pharmazeutische Präparat kann im Anschluss an Schritt (c) oder Schritt (d), falls dieser Schritt erforderlich sein sollte, sterilisiert werden. Die Sterilisation kann beispielsweise durch eine Sterilfiltration erfolgen.

Als Aminosäure mit einem isoelektrischen Punkt größer 7 wird wie bereits oben beschrieben Arginin, Lysin oder Histidin oder eine Kombination von diesen eingesetzt. Die Säure zur Einstellung des pH-Wertes wurde ebenfalls bereits oben beschrieben.

Für das erfindungsgemäße Verfahren ist es wesentlich, dass der pH-Wert ab dem Schritt (b) immer in dem Bereich von 3 bis 5 gehalten wird. Bevorzugt ist, dass der pH-Wert in dem Bereich von ca. 3,5 bis 4,5, insbesondere in dem Bereich von 3,9 bis 4,1 und besonders bevorzugt bei ca. 4,0 gehalten wird.

In einer besonders bevorzugten Ausführungsform des Verfahrens gemäß der Erfindung wird zunächst Arginin in Wasser gelöst, dann wird Asparaginsäure (vorzugsweise in ungefähr äquimolarer Menge zu der Menge an Arginin) zugegeben und gelöst, anschließend wird der pH-Wert mit Citronensäure auf ca. 3,9 bis 4,1 eingestellt, danach wird Vecuroniumbromid zugegeben und gelöst und schließlich wird der pH-Wert mit Citronensäure noch mal auf 3,9 bis 4,1 eingestellt. Die so hergestellten pharmazeutischen Präparate zeichnen sich durch eine besonders gute Haltbarkeit aus.

Die vorliegende Erfindung wird nun anhand der folgenden Beispiele näher erläutert. .

### BEISPIELE

### Beispiel 1

### 1.1 Herstellung einer Injektionslösung

Ein Ansatz von 10 Litern Injektionslösung wurde wie folgt hergestellt: 174 g (1 Mol) Arginin (erhalten von Fluka) wurden in 9.000 ml Wasser für Injektionszwecke (WfI) mithilfe eines Rührers gelöst. Anschließend wurden 133 g (1 Mol) Asparaginsäure (ebenfalls erhalten von Fluka) zugegeben, und es wurde weiter gerührt, bis sich die Asparaginsäure gelöst hatte. Anschließend wurde der pH unter Verwendung von 5%iger Citronensäure (50 g Citronensäure Monohydrat Eur. Ph., aufgefüllt auf 1000 g mit WfI) auf ca. 4,0 eingestellt. Danach wurden 40 g (0,06 Mol) Vecuroniumbromid (erhalten von Poli Industria Chimica S.p.A.) zu der Lösung zugegeben, und es wurde bis zur Lösung weiter gerührt. Danach wurde der pH wieder unter Verwendung der 5%igen Citronensäure auf ca. 4,0 eingestellt. Schließlich wurde mit WfI auf 10 Liter aufgefüllt.

Die erhaltene farblose und klare Injektionslösung wurde durch sterilisierte Membranfilter mit 0,2 µm unter Stickstoffdruck (max. 1,5 bar) filtriert und anschließend in 1 ml-Glasampullen abgefüllt. Die Injektionslösung in den Ampullen war bei ca. 5°C über ein Jahr lang haltbar.

### 1.2 Stabilität der erfindungsgemäßen Injektionslösungen im Vergleich zu Lösungen nach dem Stand der Technik

1 ml-Ampullen mit Injektionslösung, welche wie oben beschrieben in zwei Ansätzen (Charge 1 und Charge 2) hergestellt wurden, wurden bei 5°C ± 3°C im Kühlschrank gelagert. Zu Beginn sowie nach 3, 6, 9 und 12 Monaten wurden einzelne Ampullen entnommen und im Hinblick auf ihren Wirkstoffgehalt an Vecuroniumbromid mittels HPLC analysiert. Die Detektion erfolgte bei 210 nm.

Das Ergebnis der HPLC-Analyse ist in der folgenden Tabelle gezeigt. Der Wirkstoffgehalt zu Beginn (0 Monate) wurde auf 100% gesetzt.

**Tabelle**

| | Gehalt an Vecuroniumbromid | | | | |
|---|---|---|---|---|---|
| | 0 Monate | 3 Monate | 6 Monate | 9 Monate | 12 Monate |
| Charge 1 | 100% | 98,2 ± 0,4% | 97,5 ± 1,0% | 96,1 ± 1,0% | 97,4 ± 1,0% |
| Charge 2 | 100% | 98,4 ± 0,6% | 99,3 ± 1,0% | 98,4 ± 1,0% | 96,5 ± 1,2% |

Die Tabelle zeigt, dass die erfindungsgemäßen Injektionslösungen auch nach 12 Monaten Lagerung noch einen Wirkstoffgehalt von weit über 95% aufwiesen.

Die aus dem im Handel erhältlichen Vecuroniumbromidlyophilisat Norcuron^{®} hergestellte Lösung ist dagegen im Kühlschrank nur bis zu maximal 5 Tagen haltbar (vergl. Handbook on Injectable Drugs, 11. Ausgabe, 2001, L.A. Trissel, Herausgeber: ASHP).

## Patentansprüche

1. Pharmazeutisches Präparat in Form einer wässrigen Lösung, welches Vecuroniumbromid als arzneilich wirksamen Bestandteil, eine Aminosäure mit einem isoelektrischen Punkt größer 7, die ausgewählt ist aus Arginin, Lysin und Histidin, und mindestens eine Aminosäure mit einem isoelektrischen Punkt kleiner 3,5 und eine organische Säure zur Einstellung des pH-Wertes auf einen Wert in dem Bereich von 3 bis 5 umfasst,
welches erhältlich ist durch ein Verfahren, das die folgenden Schritte umfasst:
(a) Zugeben einer Aminosäure mit einem isoelektrischen Punkt größer 7, die ausgewählt ist aus Arginin, Lysin und Histidin, zu Wasser und Auflösen derselben unter Rühren,
(b) Zugeben mindestens einer Aminosäure mit einem isoelektrischen Punkt kleiner 3,5 und einer organischen Säure zur Einstellung des pH-Wertes auf einen Wert in dem Bereich von 3 bis 5 zu der Lösung aus Schritt (a) und Weiterrühren bis zu deren Auflösung,
(c) Zugeben von Vecuroniumbromid und Weiterrühren bis zu dessen Auflösung und
(d) falls erforderlich erneutes Zugeben einer Säure zur Einstellung des pH-Wertes in dem Bereich von 3 bis 5.

2. Pharmazeutisches Präparat nach Anspruch 1, **dadurch gekennzeichnet, dass** es als Aminosäure mit einem isoelektrischen Punkt kleiner 3,5 Asparaginsäure oder Glutaminsäure umfasst.

3. Pharmazeutisches Präparat nach Anspruch 1 oder Anspruch 2, **dadurch gekennzeichnet, dass** es als organische Säure Citronensäure, Essigsäure, Propionsäure, Maleinsäure, Fumarsäure, Bernsteinsäure, Glutarsäure, Weinsäure oder Milchsäure umfasst.

4. Pharmazeutisches Präparat nach Anspruch 3, **dadurch gekennzeichnet, dass** die organische Säure Citronensäure ist.

5. Pharmazeutisches Präparat nach einem der Ansprüche 1 bis 4, **dadurch gekennzeichnet, dass** es einen pH-Wert von 3,5 bis 4,5, vorzugsweise 3,9 bis 4,1 und besonders bevorzugt ca. 4,0 aufweist.

6. Pharmazeutisches Präparat nach einem der Ansprüche 1 bis 5, **dadurch gekennzeichnet, dass** Vecuroniumbromid in dem Präparat in einer Konzentration von 1 - 5 mg/ml, insbesondere 2 - 4 mg/ml vorliegt.

7. Pharmazeutisches Präparat nach einem der Ansprüche 1 bis 6, **dadurch gekennzeichnet, dass** es als Aminosäure mit einem isoelektrischen Punkt größer 7 Arginin und als Säuren zur Einstellung des pH-Wertes Asparaginsäure und Citronensäure umfasst.

8. Pharmazeutisches Präparat nach einem der Ansprüche 1 bis 7, **dadurch gekennzeichnet, dass** es in Form einer gebrauchsfertigen Lösung vorliegt.

9. Verfahren zur Herstellung eines pharmazeutischen Präparats nach einem der Ansprüche 1 bis 8, **dadurch gekennzeichnet, dass** es die folgenden Schritte umfasst:
(a) Zugeben einer Aminosäure mit einem isoelektrischen Punkt größer 7, die ausgewählt ist aus Arginin, Lysin und Histidin, zu Wasser und Auflösen derselben unter Rühren,
(b) Zugeben mindestens einer Aminosäure mit einem isoelektrischen Punkt kleiner 3,5 und einer organischen Säure zur Einstellung des pH-Wertes auf einen Wert in dem Bereich von 3 bis 5 zu der Lösung aus Schritt (a) und Weiterrühren bis zu deren Auflösung,
(c) Zugeben von Vecuroniumbromid und Weiterrühren bis zu dessen Auflösung und
(d) falls erforderlich erneutes Zugeben einer Säure zur Einstellung des pH-Wertes in dem Bereich von 3 bis 5.

10. Verfahren nach Anspruch 9, **dadurch gekennzeichnet, dass** das pharmazeutische Präparat im Anschluss an Schritt (c) oder (d) sterilisiert wird.

11. Verfahren nach Anspruch 9, **dadurch gekennzeichnet, dass** als Aminosäure mit einem isoelektrischen Punkt kleiner 3,5 Asparaginsäure oder Glutaminsäure eingesetzt wird.

12. Verfahren nach Anspruch 9 bis 11, **dadurch gekennzeichnet, dass** als organische Säure Citronensäure, Essigsäure, Propionsäure, Maleinsäure, Fumarsäure, Bernsteinsäure, Glutarsäure, Weinsäure oder Milchsäure eingesetzt wird.

13. Verfahren nach Anspruch 12, **dadurch gekennzeichnet, dass** als organische Säure Citronensäure eingesetzt wird.

14. Verfahren nach einem der Ansprüche 9 bis 13, **dadurch gekennzeichnet, dass** der pH-Wert des Präparats auf 3,5 bis 4,5, vorzugsweise 3,9 bis 4,1 und besonders bevorzugt ca. 4,0 eingestellt wird.

15. Verfahren nach einem der Ansprüche 9 bis 14, **dadurch gekennzeichnet, dass** Vecuroniumbromid in einer solchen Menge zugegeben wird, dass es in dem Präparat in einer Konzentration von 1 - 5 mg/ml; insbesondere 2 - 4 mg/ml vorliegt.

16. Verfahren nach einem der Ansprüche 9 bis 15, **dadurch gekennzeichnet, dass** als Aminosäure mit einem isoelektrischen Punkt größer 7 Arginin und als Säuren zur Einstellung des pH-Wertes Asparaginsäure und Citronensäure eingesetzt werden.

## Claims

1. Pharmaceutical preparation in form of an aqueous solution, comprising vecuronium bromide as pharmaceutically active ingredient, an amino acid having an isoelectric point greater than 7, which is selected from arginine, lysine, and histidine, and at least one amino acid having an isoelectric point smaller than 3.5 and an organic acid for adjusting the pH value to a value in the range of 3 to 5,
which is obtainable by a method comprising the following steps:
(a) adding an amino acid having an isoelectric point greater than 7, which is selected from arginine, lysine, and histidine, to water and dissolving same under stirring,
(b) adding at least one amino acid having an isoelectric point smaller than 3.5 and an organic acid for adjusting the pH value to a value in the range of 3 to 5 to the solution of step (a) and continuing stirring until its dissolution,
(c) adding vecuronium bromide and continuing stirring until its dissolution, and
(d) if necessary, adding again an acid for adjusting the pH value in the range of 3 to 5.

2. Pharmaceutical preparation according to claim 1, **characterized in that** it comprises asparaginic acid or glutamic acid as an amino acid having an isoelectric point smaller than 3.5.

3. Pharmaceutical preparation according to claim 1 or claim 2, **characterized in that** it comprises citric acid, acetic acid, propionic acid, maleic acid, fumaric acid, succinic acid, glutaric acid, tartaric acid or lactic acid as organic acid.

4. Pharmaceutical preparation according to claim 3, **characterized in that** the organic acid is citric acid.

5. Pharmaceutical preparation according to one of claims 1 to 4, **characterized in that** it has a pH value of 3.5 to 4.5, preferably of 3.9 to 4.1, and especially preferred of about 4.0.

6. Pharmaceutical preparation according to one of claims 1 to 5, **characterized in that** vecuronium bromide is present in the preparation in a concentration of 1 - 5 mg/ml, in particular 2 - 4 mg/ml.

7. Pharmaceutical preparation according to one of claims 1 to 6, **characterized in that** it comprises arginine as amino acid having an isoelectric point greater than 7 and asparaginic acid and citric acid as acids for adjusting the pH value.

8. Pharmaceutical preparation according to one of claims 1 to 7, **characterized in that** it is present in form of a solution ready for use.

9. Method for producing a pharmaceutical preparation according to one of claims 1 to 8, **characterized in that** it comprises the following steps:
(a) adding an amino acid having an isoelectric point greater than 7, which is selected from arginine, lysine, and histidine, to water and dissolving same under stirring,
(b) adding at least one amino acid having an isoelectric point smaller than 3.5 and an organic acid for adjusting the pH value to a value in the range of 3 to 5 to the solution of step (a) and continuing stirring until its dissolution,
(c) adding vecuronium bromide and continuing stirring until its dissolution, and
(d) if necessary, adding again an acid for adjusting the pH value in the range of 3 to 5.

10. Method according to claim 9, **characterized in that** the pharmaceutical preparation is sterilized after step (c) or (d).

11. Method according to claim 9, **characterized in that** asparaginic acid or glutamic acid is employed as amino acid having an isoelectric point smaller than 3.5.

12. Method according to claims 9 to 11, **characterized in that** citric acid, acetic acid, propionic acid, maleic acid, fumaric acid, succinic acid, glutaric acid, tartaric acid or lactic acid is employed as organic acid.

13. Method according to claim 12, **characterized in that** citric acid is employed as organic acid.

14. Method according to one of claims 9 to 13, **characterized in that** the pH value of the preparation is adjusted to 3.5 to 4.5, preferably to 3.9 to 4.1, and particularly preferred to about 4.0.

15. Method according to one of claims 9 to 14, **characterized in that** vecuronium bromide is added in such an amount that it is present in the preparation in a concentration of 1 - 5 mg/ml, in particular 2 - 4 mg/ml.

16. Method according to one of claims 9 to 15, **characterized in that** arginine is employed as an amino acid having an isoelectric point greater than 7, and asparaginic acid and citric acid are employed as organic acids for adjusting the pH value.

## Revendications

1. Préparation pharmaceutique sous forme d'une solution aqueuse, ladite préparation comprenant du bromure de vécuronium comme composant pharmaceutiquement actif, un acide aminé présentant un point isoélectrique supérieur à 7, choisi parmi l'arginine, la lysine et l'histidine, et moins un acide aminé présentant un point isoélectrique inférieur à 3,5 et un acide organique destiné à ajuster le pH à une valeur comprise entre 3 et 5,
laquelle peut être obtenue par un procédé comprenant les étapes suivantes :
(a) ajout d'un acide aminé présentant un point isoélectrique supérieur à 7, choisi parmi l'arginine, la lysine et l'histidine, à de l'eau et dissolution dudit acide sous agitation,
(b) ajout d'au moins un acide aminé présentant un point isoélectrique inférieur à 3,5 et d'un acide organique destiné à ajuster le pH à une valeur comprise entre 3 et 5 à la solution obtenue dans l'étape (a) et poursuite de l'agitation jusqu'à ce qu'ils se dissolvent complètement,
(c) ajout de bromure de vécuronium et poursuite de l'agitation jusqu'à ce qu'il se dissolve complètement et
(d) si nécessaire, nouvel ajout d'un acide destiné à ajuster le pH à une valeur comprise entre 3 et 5.

2. Préparation pharmaceutique selon la revendication 1, **caractérisée en ce qu'**elle comprend, en tant qu'acide aminé présentant un point isoélectrique inférieur à 3,5, de l'acide aspartique ou de l'acide glutamique.

3. Préparation pharmaceutique selon la revendication 1 ou la revendication 2, **caractérisée en ce qu'**elle comprend, en tant qu'acide organique, de l'acide citrique, de l'acide acétique, de l'acide propionique, de l'acide maléique, de l'acide fumarique, de l'acide succinique, de l'acide glutarique, de l'acide tartrique ou de l'acide lactique.

4. Préparation pharmaceutique selon la revendication 3, **caractérisée en ce que** ledit acide organique est l'acide citrique.

5. Préparation pharmaceutique selon l'une des revendications 1 à 4, **caractérisée en ce qu'**elle présente un pH compris entre 3,5 et 4,5, de préférence entre 3,9 et 4,1 et, avec une préférence particulière, d'environ 4,0.

6. Préparation pharmaceutique selon l'une des revendications 1 à 5, **caractérisée en ce qu'**elle contient le bromure de vécuronium dans une concentration comprise entre 1 et 5 mg/ml, notamment entre 2 et 4 mg/ml.

7. Préparation pharmaceutique selon l'une des revendications 1 à 6, **caractérisée en ce qu'**elle comprend, en tant qu'acide aminé présentant un point isoélectrique supérieur à 7, de l'arginine et, en tant qu'acides destinés à ajuster le pH, de l'acide aspartique et de l'acide citrique.

8. Préparation pharmaceutique selon l'une des revendications 1 à 7, **caractérisée en ce qu'**elle se présente sous forme d'une solution prête à l'emploi.

9. Procédé de réalisation d'une préparation pharmaceutique selon l'une des revendications 1 à 8, **caractérisé en ce qu'**il comprend les étapes suivantes
(a) ajout d'un acide aminé présentant un point isoélectrique supérieur à 7, choisi parmi l'arginine, la lysine et l'histidine, à de l'eau et dissolution dudit acide sous agitation,
(b) ajout d'au moins un acide aminé présentant un point isoélectrique inférieur à 3,5 et d'un acide organique destiné à ajuster le pH à une valeur comprise entre 3 et 5 à la solution obtenue dans l'étape (a) et poursuite de l'agitation jusqu'à ce qu'ils se dissolvent complètement,
(c) ajout de bromure de vécuronium et poursuite de l'agitation jusqu'à ce qu'il se dissolve complètement et
(d) si nécessaire, nouvel ajout d'un acide destiné à ajuster le pH à une valeur comprise entre 3 et 5.

10. Procédé selon la revendication 9, **caractérisé en ce que** la préparation pharmaceutique est stérilisée suite à l'étape (c) ou l'étape (d).

11. Procédé selon la revendication 9, **caractérisé en ce qu'**on met en oeuvre, en tant qu'acide aminé présentant un point isoélectrique inférieur à 3,5, de l'acide aspartique ou de l'acide glutamique.

12. Procédé selon les revendications 9 à 11, **caractérisé en ce qu'**on met en oeuvre, en tant qu'acide organique, de l'acide citrique, de l'acide acétique, de l'acide propionique, de l'acide maléique, de l'acide fumarique, de l'acide succinique, de l'acide glutarique, de l'acide tartrique ou de l'acide lactique.

13. Procédé selon la revendication 12, **caractérisé en ce qu'**on met en oeuvre, en tant qu'acide organique, l'acide citrique.

14. Procédé selon l'une des revendications 9 à 13, **caractérisé en ce qu'**on ajuste le pH de la préparation à une valeur comprise entre 3,5 et 4,5, de préférence entre 3,9 et 4,1 et, avec une préférence particulière, d'environ 4,0.

15. Procédé selon l'une des revendications 9 à 14, **caractérisé en ce qu'**on ajoute le bromure de vécuronium dans quantité telle que sa concentration dans la préparation est comprise entre 1 et 5 mg/ml, notamment entre 2 et 4 mg/ml.

16. Procédé selon l'une des revendications 9 à 15, **caractérisé en ce qu'**on met en oeuvre, en tant qu'acide aminé présentant un point isoélectrique supérieur à 7, de l'arginine et, en tant qu'acides destinés à ajuster le pH, de l'acide aspartique et de l'acide citrique.
